(19) Europäisches Patentamt
European Patent Office
Office européen des brevets

(11) Publication number: **0 107 938 A1**

(12) **EUROPEAN PATENT APPLICATION**

(21) Application number: 83306052.8

(22) Date of filing: 05.10.83

(51) Int. Cl.³: C 07 D 215/20
C 07 D 215/22, C 07 D 215/26
C 07 D 215/48, C 07 D 215/50
C 07 D 215/54, C 07 D 215/56
A 61 K 31/47
//C07D407/12, C07D215/32

(30) Priority: 05.10.82 JP 173950/82

(43) Date of publication of application: 09.05.84 Bulletin 84/19

(84) Designated Contracting States: DE FR GB IT SE

(71) Applicant: KOWA COMPANY, LTD.
6-29, 3-chome Nishiki
Naka-ku Nagoya-shi Aichi-ken(JP)

(72) Inventor: Shiratsuchi, Masami
4-43-2, Zanbori
Musashimurayama-shi Tokyo(JP)

(72) Inventor: Onogi, Kazuhiro
457-19, Ohgimachiya
Iruma-shi Saitama-ken(JP)

(72) Inventor: Sato, Seiichi
1-6-77, Honcho
Higashiurayama-shi Tokyo(JP)

(72) Inventor: Ishihama, Hiroshi
2-8-8, Suwa-cho
Higashimurayama-shi Tokyo(JP)

(74) Representative: Myerscough, Philip Boyd et al,
J.A.Kemp & Co. 14, South Square Gray's Inn
London, WC1R 5EU(GB)

(54) **Quinoline or hydroquinoline compounds, their preparation and pharmaceutical compostions comprising them.**

(57) A substituted quinoline or hydroquinoline compound of formula (I)

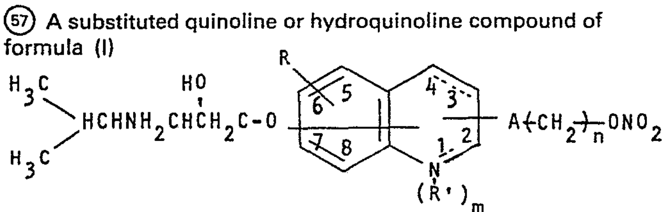

(I)

wherein

R represents a hydrogen atom or a $CF_3$ group,

A represents a direct bond or the group -CONH-,

R' represents a hydrogen atom or an alkoxy-carbonyl group,

n represents an integer of 1 to 4, and

m represents 0 or 1, provided that when m is 0, the bond between the 1-position nitrogen atom and the 2-position carbon atom and the bond between the 3-position carbon atom and the 4-position carbon atom are both double bonds, and when m is 1, the two bonds are both single bonds, or a pharmaceutically acceptable acid addition salt thereof; a process for producing the aforesaid compound; and a pharmaceutical composition comprising the same.

EP 0 107 938 A1

DESCRIPTION

TITLE: "QUINOLINE OR HYDROQUINOLINE COMPOUNDS, THEIR PREPARATION AND PHARMACEUTICAL COMPOSITIONS COMPRISING THEM"

This invention relates to novel quinoline or hydroquinoline compound having various pharmacological activities such as blood pressure lowering activity, vasodilating activity, adrenergic β-blocking activity and blood flow increasing activity. The invention also relates to a process for producing the aforesaid compounds.

The compounds of this invention have not been described in the literature and, therefore, are novel. They can be represented by the following formula (I).

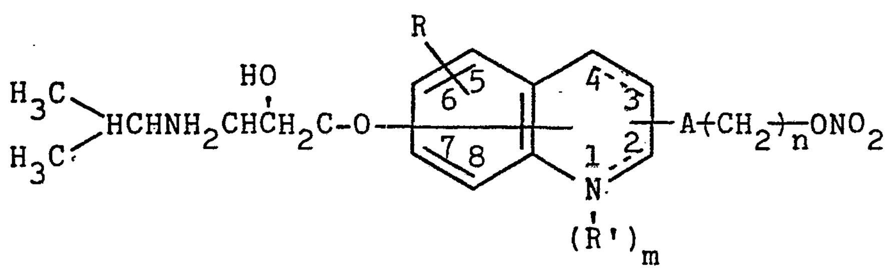

(I)

In the formula, R represents a hydrogen atom or a $CF_3$ group, A represents a direct bond or the bond -CONH-, R' represents a hydrogen atom or a lower alkoxycarbonyl group, n represents an integer of 1 to 4, and m is 0 or 1, provided that when m is 0, the bond between the 1-position nitrogen atom and the 2-position carbon atom and the bond between the 3-position carbon atom and the 4-position carbon atom are both double bonds, and when m is 1, the two bonds are single bonds.

Some workers including some of inventors of the present application previously proposed benzopyran compounds of the following formula

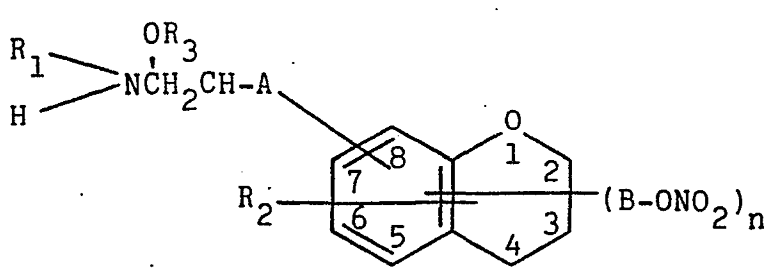

wherein

A represents a direct bond or the bond $-CH_2-O-$, $R_1$ represents a member selected from the group consisting of a $C_3$-$C_5$ alkyl group, a hydroxy-($C_3$-$C_5$ alkyl) group, a lower alkylamino-lower alkyl group, a nitrato-($C_3$-$C_5$ alkyl) group and a phenyl-($C_1$-$C_5$ alkyl) group, provided that the phenyl may be substituted by a lower alkoxy group,

$R_2$ represents a member selected from the group consisting of hydrogen, halogen, OH, $NO_2$, a carbamoyl group, a lower alkyl group, a lower alkoxy group, a lower alkyleneoxy group and an acetyl group,

$R_3$ represents hydrogen or $NO_2$, B represents a direct bond, a $C_1$-$C_7$ alkylene group, a -O-lower alkylene group or a -CONH-lower alkylene group, and

n represents 1 or 2,

which have various pharmacological activities such as vascular smooth muscle relaxing action, adrenergic α- and β-blocking action resulting in a reduction in heart beat rate, myocardial oxygen consumption reducing action, blood flow increasing action and blood pressure lowering action (European Laid-Open Patent Publication No. 0034461 laid-open on August 26, 1981; corresponding to U. S. Patent No. 4,394,382).

The present inventors have now succeeded in synthesizing the quinoline or hydroquinoline compounds of formula (I) having a different ring skeletal structure from the above benzopyran compounds. These compounds of formula (I) in accordance with present invention have been found to have various pharmacological activities such as blood pressure lowering activity, vasodilating activity, adrenergic β-blocking activity, and blood flow increasing activity.

It is an object of this invention therefore

to provide novel quinoline or hydroquinoline compounds of formula (I) and a process for producing them.

In the compounds of formula (I) in accordance with this invention, the (2-hydroxy-3-isopropylamino)-propyloxy group of the formula

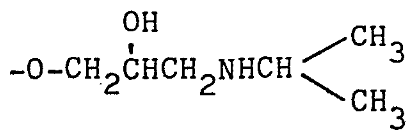

may be bonded to any of the 1- to 8-positions of formula (I), preferably to the 4-, 5- or 8-position. The group R may be bonded to any of the 5- to 8-positions. The group $-A\!\leftarrow\!CH_2\!\rightarrow_n\!ONO_2$ may be bonded to any of the 2- to 4-positions, preferably to the 2- or 3-position. The lower alkoxycarbonyl group for the group R' is preferably an alkoxycarbonyl group having 1 to 3 carbon atoms in the alkyl moiety, particularly a methoxycarbonyl group.

Specific examples of the compound of formula (I) are as follows:

4-, 5-, or 8-[(2-hydroxy-3-isopropylamino)-propyloxy]-N-(2- or 3-nitroxypropyl)quinoline-2- or 3-carboxamide,

4-, 5-, or 8-[(2-hydroxy-3-isopropylamino)-propyloxy]-N-(2- or 3-nitroxypropyl)-7-trifluoro-methylquinoline-2- or 3-carboxamide,

1-[2-nitroxymethyl-5-quinolyloxy]-3-isopropyl-amino-2-propanol,

1-methoxycarbonyl-2- or 3-nitroxymethyl-1,2,3,4-tetrahydro-8-[(3-isopropylamino-2-hydroxy)-propyloxy]quinoline,

2- or 3-nitroxymethyl-1,2,3,4-tetrahydro-8-[(3-isopropylamino-2-hydroxy)propyloxy]quinoline, and

the pharmaceutically acceptable acid addition salts of the above-exemplified compounds.

The compounds of formula (I) in accordance with this invention can be produced, for example, by the following process (a) or (b).

Process (a)

A compound of the following formula (II)

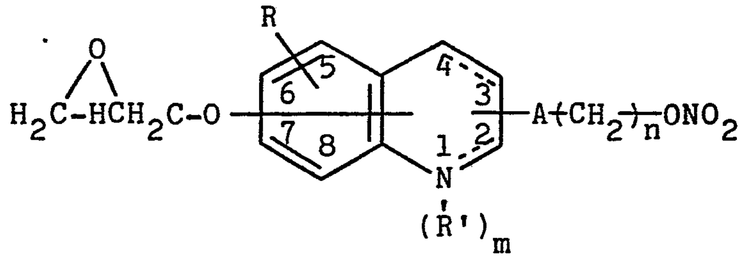

. (II)

wherein R, A, R', n and m are as defined above,

is reacted with isopropylamine.

Process (b)

A compound of the following formula (III)

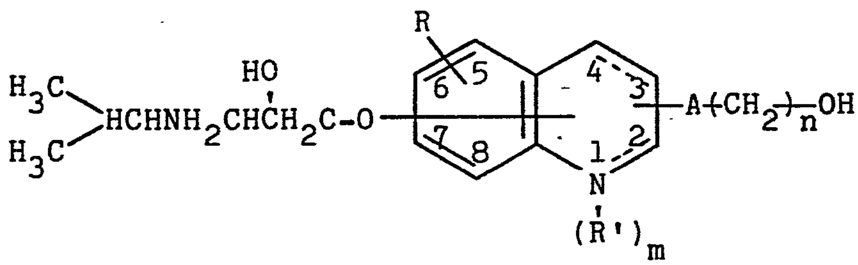

(III)

wherein R, A, R', n and m are as defined above,

is subjected to a nitrate-ester-forming reaction.

In performing the process (a) or (b), when m in the starting compound of formula (II) or (III) is 1 and R' is a hydrogen atom, it is preferred to subject such a tetrahydroquinoline compound to the reaction in process (a) or (b) after the N atom in the tetrahydroquinoline ring is protected by a protective group, for example a lower alkoxycarbonyl group such as those exemplified with respect to the group R', or a benzyloxycarbonyl group.

This protection can be carried out, for example, by strongly stirring the tetrahydroquinoline compound in water, dioxane or tetrahydrofuran at room temperature to 80°C in the presence or the corresponding halogenoformate and a strong base such as sodium hydroxide, sodium carbonate, sodium bicarbonate or $NEt_3$.

Amination with isopropylamine in process (a) can be carried out, for example, by contacting the compound of formula (II) with isopropylamine in a suitable solvent. The solvent which can be used at this time is an inert organic solvent such as methanol, ethanol or isopropanol. The reaction is carried out preferably under heating, for example under refluxing conditions. A reaction temperature of, for example, about 70 to about 90°C may be employed. The reaction time can be properly selected, and is, for example, 0.5 to 5 hours.

The nitrate ester-forming reaction of process (b) can be carried out by contacting the compound of formula (III) with a nitrating agent such as fuming nitric acid or a mixture of it with acetic anhydride in the presence or absence of a solvent. Examples of the solvent which may be used are acetonitrile, dioxane and tetrahydrofuran. Preferably, the reaction is carried out at relatively low temperatures, for example about -40°C to room temperature. The reaction time can be properly selected, and is, for example, several minutes to 3 hours.

The starting compound of formula (II) in process (a) can be produced, for example, by subjecting a compound of the following formula

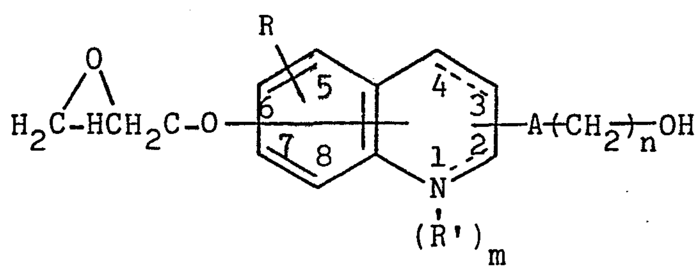

(IV)

wherein R, A, R', n and m are as defined above,

to a nitrate ester-forming reaction. The reaction can be carried out in the same way as in process (b) except that the compound of formula (IV) is used instead of the compound of formula (III).

The starting compound of formula (III) used in process (b) can be produced by reacting the compound of formula (IV) with isopropylamine. Amination with isopropylamine can be carried out in the same way as in process (a) except that the compound of formula (IV) is used instead of the compound of formula (II).

The compound of formula (IV) can be produced by a method known per se. For example, it can be produced by reacting a hydroxyquinoline carboxylic acid of formula (IV) in which A is the bond -CONH- with a hydroxyalkylamine $H_2N{+}CH_2{+}_nOH$ wherein n is as defined above and an epihalohydrin.

The amidation reaction with the hydroxyalkylamine and the epoxidation reaction with the epihalohydrin can be carried out, for example as follows.

The hydroxyquinolinecarboxylic acid ester is reacted with an excess (2 to 10 equivalents) of the hydroxylalkylamine at 80 to 150$^{o}$C for 1 to 10 hours in accordance with the following reaction scheme.

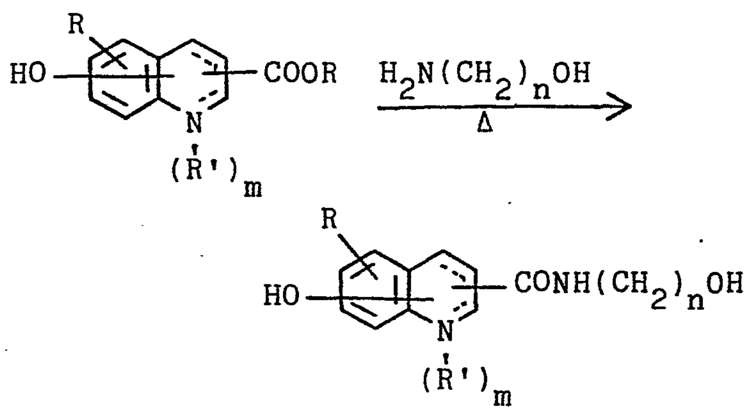


Then, the hydroxyquinoline is dissolved in an inert solvent such as methanol, ethanol, acetone, tetrahydrofuran or dioxane, and after adding a base such as sodium hydroxide, potassium hydroxide, potassium carbonate, sodium carbonate, $NEt_3$ or DBU, reacted with an excess (2 to 10 equivalents) of an epihalohydrin at room temperature to 90$^{o}$C for 1 to 20 hours as schematically shown below.

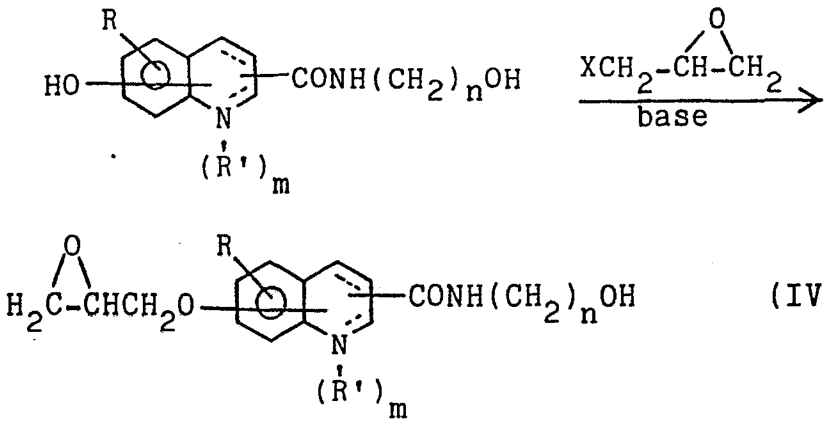


(IV)

Furthermore, a compound of formula (IV) in which A is a direct bond can be produced, for example, by reducing the hydroxyquinoline carboxylic acid and reacting the product with an epihalohydrin. This reaction can be carried out, for example, as follows:-

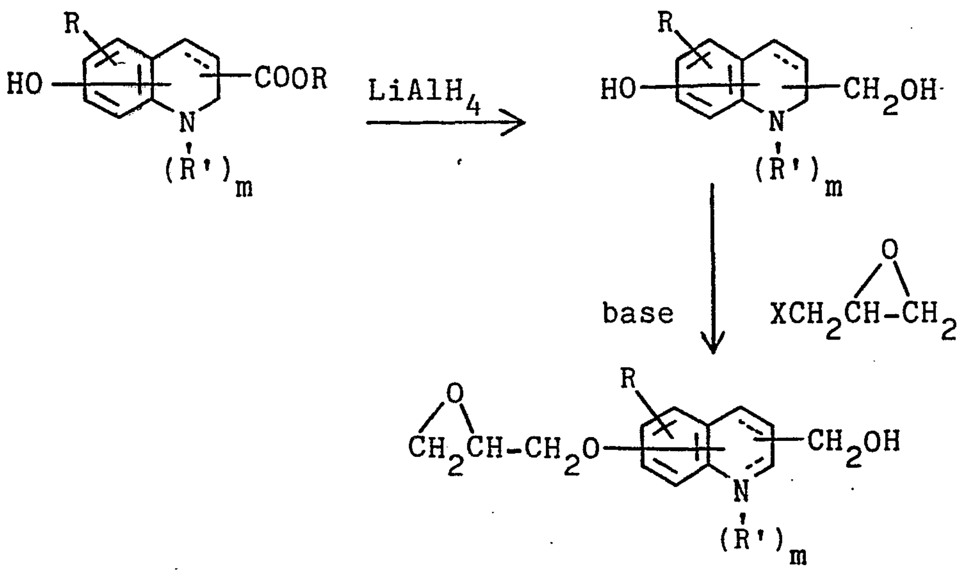


(IV)

The hydroxyquinoline carboxylicacid in reduced with a reducing agent such as lithium aluminum hydride at room temperature to $80^{o}C$ in a solvent such as ether or tetrahydrofuran, and then converted to a glycidyl ether by the method described above.

The novel compounds of formula (I) in accordance with this invention which can be produced as above can be in the form of their pharmaceutically acceptable acid addition salts.

The acid addition salt can be easily obtained by contacting the compound of formula (I) with a suitable inorganic or organic acid. Those acid

addition salts which are pharmaceutically acceptable are preferred. Examples of the acid addition salts are hydrochlorides, nitrates, sulfates, phosphates, oxalates, maleates, methanesulfonates, ethanesulfonates, p-toluenesulfonates, fumarates, lactates, malonates and acetates.

The compounds of formula (I) and their pharmaceutically acceptable acid addition salts exhibit pharmacological activities such as blood pressure lowering activity, vasodilating activity, adrenergic β-blocking activity and blood flow increasing activity, and are useful, for example, as anti-angina pectoris agents, hypotensive agents, cerebral circulation improvers and antiarrhythmic agents.

Accordingly, there can be provided in accordance with this invention a pharmaceutical composition comprising an amount, effective for treatment of cardiovascular diseases, of a compound of formula (I) and a pharmaceutically acceptable diluent or carrier.

Liquid or solid carriers or diluents may be used in forming the pharmaceutical composition. They may include excipients, binders, lubricants, emulsifiers, etc. known in pharmaceutical production. Examples of these carriers or diluents include starches such as potato starch, wheat starch, corn starch and rice starch; sugars such as lactose, sucrose, glucose, mannitrol and sorbitol; celluloses such as crystalline cellulose, calcium carboxymethyl cellulose and hydroxypropyl cellulose of a low degree of substitution; inorganic substances such as potassium phosphate, calcium sulfate, calcium carbonate and talc; binder compounds such as gelatin, gum arabic, methyl cellulose, sodium carboxymethyl cellulose, polyvinyl pyrrolidone and hydroxypropyl cellulose; polyhydric alcohol ester-type non-ionic surfactants such as fatty acid monoglycerides, sorbitan fatty acid esters, sucrose and

polyglycerol fatty acid esters; and polyoxyethylene-type nonionic surfactants.

The pharmaceutical compositions may be in any dosage forms known in the art of formulating pharmaceuticals, such as suppositories, powders, granules, tablets, sublingual tablets, liquid preparations, injectable preparations, and suspensions.

The pharmaceutical composition may be administered through any of peroral or parenteral routes, such as intravenous, sublingual or intrarectal administration. For long-term administration, the oral route is preferred.

The dose may be changed as desired. For example, the compound of formula (I) may be administered in a dose of about 1 to about 100 mg/body/day, preferably about 5 to about 50 mg/body/day. The compounds of this invention have low toxicity.

The compounds of this invention have been found to be active in the following pharmacological activity tests.

Test 1. Blood pressure and Heart rate using hypertensive rats.

Test 2. β-Blocking action using isolated atrial muscles of guinea pigs.

The following Examples illustrate the compounds of this invention and their production.

Example 1.

4-[(2-hydroxy-3-isopropylamino)propyloxy]-N-(nitroxypropyl)quinoline-2-carboxamide:-

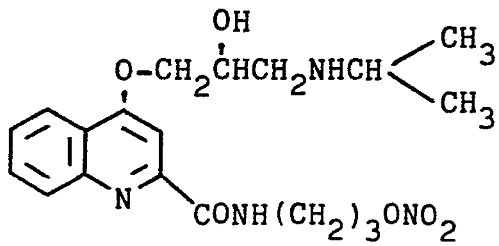

(1) 5.74g of 5-aminopropanol was added to 4.14g of ethyl 4-hydroxyquinoline-2-carboxylate, and the mixture was heated at 120°C for 3 hours. After cooling, water was added and the reaction mixture was neutralized with concentrated hydrochloric acid to form a precipitate. Methanol was added and the precipitate was dissolved under heat. The solution was left to stand overnight to give 4.04g (yield 85.9%) of 4-hydroxy-N-(3-hydroxypropyl)quinoline-2-carboxamide as pale yellow foliar crystals having a melting point of 258 to 259°C.

(2) 3.90g of the resulting compound was suspended in 100 ml of methanol, and 4.84g of 1,8-diazabicyclo[5.4.0]undec-7-ene (to be abbreviated as DBU) was added to form a uniform solution. Epichlorohydrin (14.71g) was added to the solution, and the mixture was stirred under reflux for 3 hours. After the reaction, the solvent was evaporated, and an aqueous sodium chloride solution was added to the resulting oily product, followed by extraction with chloroform. The extract was washed with an aqueous sodium chloride solution and dried. The solvent was evaporated, and 50 ml of chloroform and 4.84g of DBU were added to the resulting oily product, and the mixture was stirred under reflux for 3 hours.

After the reaction, the reaction mixture was worked up in the same way as above to give 3.26g (yield 67.9%) of 4-(2,3-epoxypropyloxy)-N-(3-hydroxypropyl)-quinoline-2-carboxamide.

MS: m/e

302($M^+$)

$^1$H-NMR: $\delta CDCl_3$

8.54 (1 H, broad.s, -N$\underline{H}$-),
8.30 (1 H, d, J=7.3Hz, HA),
8.02 (1 H, d, J=8.1Hz, HE),
7.80-7.55 (3 H, m, HB, BC, HD),
4.68, 4.63 (1 H, dd, J=2.5Hz, J=11.2Hz, -$\underline{CH_2}$-CH-$CH_2$), 4.23, 4.18 (1 H, dd, J=6.4Hz, J=4.3Hz, -$\underline{CH_2}$-CH-$CH_2$),
3.79-3.62 (4 H, m, -$\underline{CH_2}CH_2\underline{CH_2}$-),
3.57-3.49 (1 H, m, -$\underline{CH}$-$CH_2$), 3.01 (1 H, t, J=4.9Hz, -CH-$\underline{CH_2}$), 2.88, 2.86 (1 H, dd, J=2.4Hz, -CH-$CH_2$), 1.86 (2 H, q, J=5.9Hz, -$CH_2\underline{CH_2}CH_2$-).

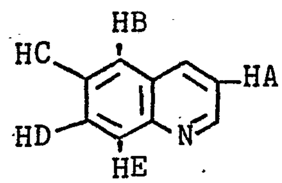

(3) To a solution of 0.50g of the resulting epoxy-amide compound in 6 ml of acetonitrile was added dropwise 6 ml of a mixture of acetic anhydride and fuming nitric acid, and the mixture was stirred for 2 hours. After the reaction, an aqueous solution of sodium bicarbonate was added to the reaction mixture, and the mixture was extracted with ethyl acetate. The extract was washed with an aqueous solution of sodium bicarbonate, dried and purified by silica gel column chromatography to give 0.150g (yield 26.0%) of 4-(2,3-epoxypropyloxy)-N-(nitroxypropyl)quinoline-2-carboxamide as a colorless oil.

M/S: m/e

347($M^+$)

$^1$H-NMR: $\delta CDCl_3$

8.53 (1 H, t, -CONH-), 8.30, 8.26 (1 H, dt, J=1.0Hz, HE), 8.06, 8.02 (1 H, dt, J=0.6Hz, HB), 7.76 (1 H, tt, J=6.8Hz, J=1.5Hz, HD), 4.67, 4.61 (1 H, dd, J=2.4Hz, 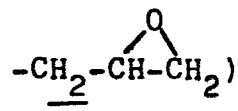), 4.60 (2 H, t, J=6.3Hz, $-CH_2ONO_2$), 4.23, 4.17 (1 H, dd, J=6.1Hz, 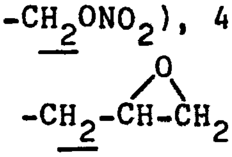), 3.65 (2 H, q, J=6.4Hz, $-NHCH_2CH_2-$), 3.59-3.50 (1H, m, 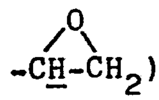), 3.02, 2.99 (1 H, dd, J=4.2Hz, 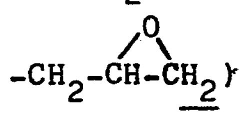), 2.87, 2.85 (1 H, dd, J=2.7Hz, 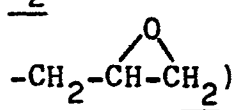), 2.14 (2 H, q, J=6.3Hz, $-CH_2CH_2CH_2-$).

(4) 0.225g of isopropylamine was added to a solution of 0.132g of the resulting epoxynitroxy compound in 5 ml of methanol, and the mixture was stirred for 3 hours under reflux. After the reaction, the solvent was evaporated and the resulting yellow oil was crystallized from ethyl acetate to give 0.158g (quantitative) of 4-[(2-hydroxy-3-isopropylamino)propyloxy]-N-(nitroxypropyl)quinoline-2-carboxamide.

IR: $\nu_{max}^{KBr}$ $cm^{-1}$

3380 (-OH), 3280 (-NH), 1680 (-CONH), 1620, 1280 ($-ONO_2$).

$^1$H-NMR: $\delta CDCl_3$

8.48 (1H, t, j=8.0Hz, -CONH-), 8.25 (1H, d, J=8.3Hz, HE), 8.03 (1H, d, J=7.8Hz, HB), 7.75 (1H, t, J=8.6Hz, HC), 7.68 (1H, s, HA), 7.58 (1H, t, J=6.7Hz, HD),

4.61 (2H, t, J=6.3Hz, $-CH_2ONO_2$), 4.32 (2H, d, J=4.9Hz, $-OCH_2-\overset{OH}{C}H-$), 4.28-4.14 (1 H, m, $-\overset{OH}{C}H-$), 3.66 (2H, q, J=6.3Hz, $-NHCH_2CH_2CH_2-$), 3.07-2.77 (3H, m, $-CH_2NHCH<$), 2.15 (2H, q, J=6.6Hz, $-CH_2CH_2CH_2-$), 2.25-1.75 (2H, broad, -OH, -NH-), 1.13 (6H, d, J=6.4Hz, $-CH<^{CH_3}_{CH_3}$).

Example 2

In the same way as in Example 1, 4-[(2-hydroxy-3-isopropylamino)propyloxy]-N-(3-nitroxypropyl)-7-trifluoromethylquinoline-3-carboxamide of the following formula was obtaind.

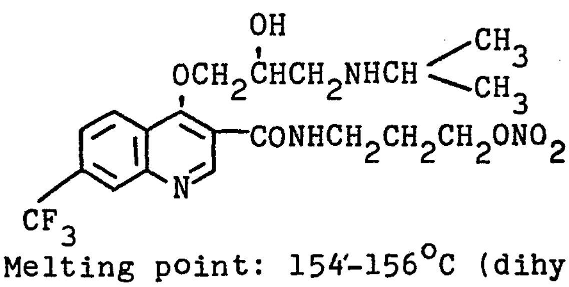

Melting point: 154-156°C (dihydrochloride)

Property: Colorless prisms

IR: $\nu_{max}^{KBr}$ $cm^{-1}$

3280 (-NH-), 1620 (-CONH, $-ONO_2$), 1280 ($-ONO_2$).

$^1$H-NMR: $\delta CD_3OD$

8.92 (1 H, s, HD), 8.63 (1 H, d, J=8.3Hz, HC), 8.27 (1 H, s, HA), 7.81 (1 H, d, J=7.3Hz, HB), 4.90-4.74 (1 H, m, $-\overset{OH}{C}H-$), 4.60 (2H, t, J=6.4Hz, $-CH_2ONO_2$), 4.46-4.24 (2H, m, $-OCH_2-$), 3.67-3.11 (5H, m, $-CONHCH_2-$, $-CH_2NHCH<$), 2.06 (2H, q, J=6.4Hz, $-CH_2CH_2CH_2-$), 1.39 (6H, d, J=6.6Hz, $-CH<^{CH_3}_{CH_3}$).

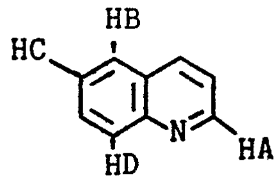

**Example 3**

In the same way as in Example 1, 1-[(2-nitroxymethyl-5-quinolyl)oxy]-3-isopropylamino-2-propanol of the following formula was obtained.

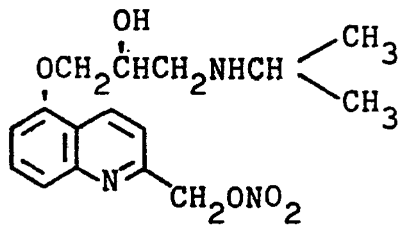

Property: Amorphous solid

IR: $\nu_{max}^{CHCl_3}$ $cm^{-1}$

1640 ($-ONO_2$)

NMR: $\delta CDCl_3$

8.80-6.70 (5H, m, aromatic ring H), 5.73 (2H, s, $-CH_2ONO_2$), 1.20 (6H, d, J=6.0Hz, -CH 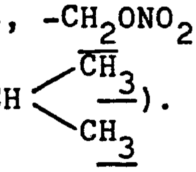).

**Example 4**

1-Methoxycarbonyl-3-nitroxymethyl-1,2,3,4-tetrahydro-8-[(3-isopropylamino-2-hydroxy)propoxy]quinoline of the following formula:-

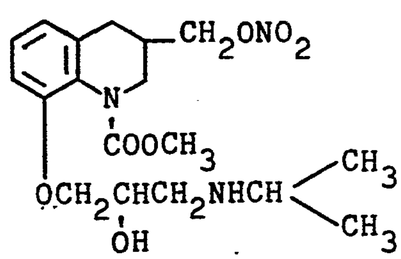

(1) A suspension of 3.0g of lithium aluminum hydride in 200 ml of anhydrous ether was cooled to below 0°C, and a solution of 9.0g of methyl 1-benzyloxycarbonyl-3-hydroxy-1,2,3,4-tetrahydro-3-quinolinecarboxylate in 200 ml of anhydrous ether was added dropwise. After the addition, the mixture was stirred

for 30 minutes and then hydrous ether was added. Furthermore, dilute hydrochloric acid was added, and the mixture was filtered. The filtrate was extracted with ethyl acetate, and the extract was washed with an aqueous sodium chloride solution and dried. The solvent was then evaporated to give 5.5g of 1-benzyloxycarbonyl-8-hydroxy-1,2,3,4-tetrahydro-3-quinolinemethanol.

A mixture consisting of 4.8g of the resulting compound, 864 ml of triethylamine and 72 ml of tetrahydrofuran was cooled, and ethyl chloroformate was added dropwise. The mixture was then stirred to perform reaction. The reaction mixture was filtered, and the solvent was evaporated to give 5.92g of a crude product. The crude product was chromatographed on a column of silica gel using chloroform as an eluent to give 3.68g of 1-benzyloxycarbonyl-8-ethoxycarbonyloxy-1,2,3,4-tetrahydro-3-quinolinemethanol.

(2) A solution of 3.36g of the resulting quinoline methanol in 70 ml of acetonitrile was maintained at below $0^{o}C$, and a mixture of 1.35g of acetic anhydride and 824 mg of fuming nitric acid was added dropwise. Fifteen minutes later, this mixture was further added freshly. After the reaction, an aqueous solution of sodium bicarbonate and ice water were added, and the mixture was extracted with ethyl acetate. The ethyl acetate layer was separated and dried. The solvent was evaporated, and the resulting oily product was purified by silica gel column chromatography to give 1.56g of 1-benzyloxycarbonyl-8-ethoxycarbonyloxy-1,2,3,4-tetrahydro-3-quinolinemethylnitrate.

IR: $\nu_{max}^{liquid\ film}$ $cm^{-1}$

1760 ($-OCOOC_2H_5$), 1700 ($>N-COOCH_2-$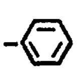 ),

1620 ($-ONO_2$).

NMR: $\delta CDCl_3$

7.45-6.95 (8H, m, aromatic ring H),

5.17 (2H, s, $-COOCH_2-$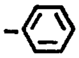), 4.25 (2H, d, J=6.0Hz, $-CH_2ONO_2$), 1.33 (3H, t, J=6.0Hz, $-OCH_2CH_3$).

(3) A solution of 1.35g of the resulting methylnitrate in 50 ml of methanol was cooled with ice, and with stirring, 3.5 ml of an 1N-sodium hydroxide solution was added. After the reaction, acetic acid was added to neturalize the reaction mixture. The solvent was then evaporated, and the residue was dissolved in ethyl acetate washed with an aqueous sodium chloride solution and dried. The solvent was then evaporated to give 1.12g of 1-benzyloxycarbonyl-3-nitroxymethyl-1,2,3,4-tetrahydro-8-quinolinol.

A mixture consisting of 930 mg of the resulting compound, 1.77g of epibromohydrin, 894 mg of potassium carbonate and 12 ml of methanol was stirred at 60°C for 2 hours. After the reaction, the solvent was evaporated, and dilute hydrochloric acid and ethyl acetate were added. The ethyl acetate layer was then separated, washed with an aqueous sodium chloride solution and dried. The solvent was evaporated to give an oily product. The oily product was purified by silica gel column chromatography to give 264 mg of 8-(2,3-epoxypropoxy)-1-methoxycarbonyl-1,2,3,4-tetrahydro-3-quinolinemethyl nitrate.

IR: $\nu_{max}^{liquid\ film}$ $cm^{-1}$

1680 ($>NCOOCH_3$), 1620 ($-ONO_2$).

NMR: $\delta CDCl_3$

7.50-6.70 (3H, m, aromatic ring H), 4.42 (2H, d, J=6.0Hz, $-CH_2ONO_2$), 3.77 (3H, s, $>N-COOCH_3$).

(4) Ten milliliters of isopropylamine was added to a solution of 220 mg of the resulting epoxy compound in 25 ml of ethanol, and the mixture was heated under reflux for 20 minutes. After reaction, the solvent was evaporated, and the residue was purified by alumina

column chromatography to give 175 mg (yield 67.7%) of 1-methoxycarbonyl-3-nitroxymethyl-1,2,3,4-tetrahydro-8-[(3-isopropylamino-2-hydroxy)propoxy]quinoline.

IR: $\nu_{max}^{liquid\ film}$ $cm^{-1}$

1690 (>$NCOOCH_3$), 1620 ($-ONO_2$).

NMR: $\delta CDCl_3$

7.40-6.70 (3H, m, aromatic ring H), 4.43 (2H, d, J=6.0Hz, $-CH_2ONO_2$), 3.80 (3H, s, $-NCOOCH_3$), 1.08 (6H, d, J=6.0Hz,

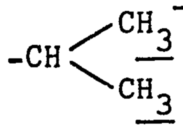 ).

Example 5

In the same way as in Example 4, 3-nitroxymethyl-1,2,3,4-tetrahydro-8-[(3-isopropylamino-2-hydroxy)propoxy]quinoline of the following formula was obtained.

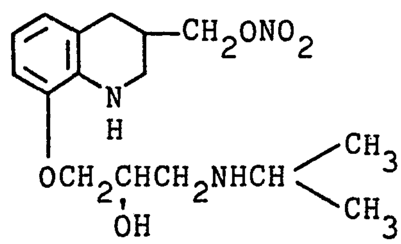

Property: Oil

IR: $\nu_{max}^{liquid\ film}$ $cm^{-1}$

1620 ($-ONO_2$)

NMR: $\delta CDCl_3$

7.50-6.70 (3H, m, aromatic ring H), 4.33 (2H, d, J=6.0Hz, $-CH_2ONO_2$), 1.06 (6H, d, J=6.0Hz, 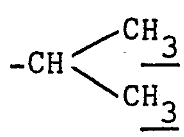 ).

CLAIMS

1. A substituted quinoline or hydroquinoline compound of formula (I)

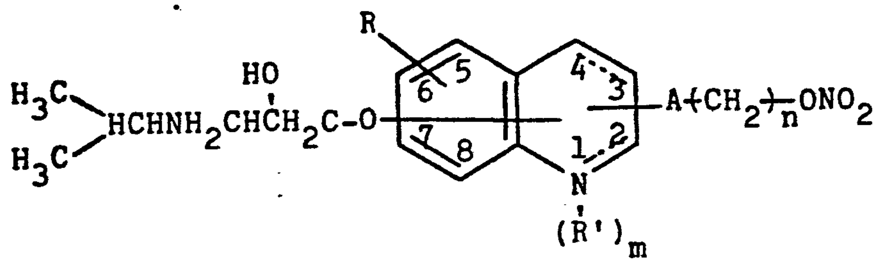

(I)

wherein

R represents a hydrogen atom or a $CF_3$ group,

A represents a direct bond or the group -CONH-,

R' represents a hydrogen atom or a lower alkoxycarbonyl group,

n represents an integer of 1 to 4, and

m represents 0 or 1, provided that when m is 0, the bond between the 1-position nitrogen atom and the 2-position carbon atom and the bond between the 3-position carbon atom and the 4-position carbon atom are both double bonds, and when m is 1, the two bonds are both single bonds; or a pharmaceutically acceptable acid addition salt thereof.

2. A compound according to claim 1 which is 4-[(2-hydroxy-3-isopropylamino)propyloxy]-N-(2-nitroxypropyl)quinoline-2-carboxamide, or a pharmaceutically acceptable acid addition salt thereof.

3. A compound according to claim 1 which is 4-[(2-hydroxy-3-isopropylamino)propyloxy]-N-(3-nitroxypropyl)-7-trifluoromethylquinoline-3-carboxamide, or a pharmaceutically acceptable acid addition salt thereof.

4. A compound according to claim 1 which is 1-[(2-nitroxymethyl-5-quinolyloxy]-3-isopropylamino-2-propanol, or a pharmaceutically acceptable acid addition salt thereof.

5. A compound according to claim 1 which is 1-methoxycarbonyl-3-nitroxymethyl-1,2,3,4-tetrahydro-8-[(3-isopropylamino-2-hydroxy)propyloxy] tetrahydroquinoline, or a pharmaceutically acceptable acid addition salt thereof.

6. A compound according to claim 1 which is 3-nitroxymethyl-1,2,3,4-tetrahydro-8-[(3-isopropyl-amino-2-hydroxy)propyloxy]quinoline, or a pharmaceutically acceptable acid addition salt thereof.

7. A process for the production of a substituted quinoline or hydroquinoline compound or a pharmaceutically acceptable acid addition salt thereof as claimed in claim 1, which process comprises

(a) reacting a compound of formula (II)

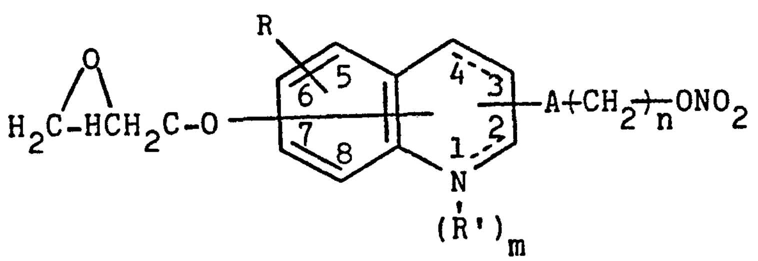

(II)

wherein R, A, R', n and m are as defined in claim 1, with isopropylamine, or

(b) subjecting a compound of formula (III)

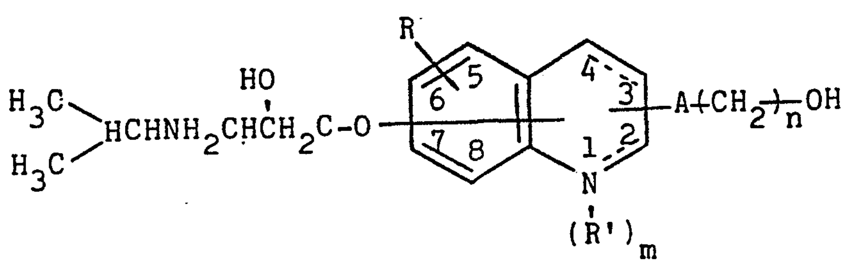

(III)

wherein R, A, R', n and m are as defined in claim 1,
to a nitrate ester-forming reaction.

8. A pharmaceutical composition comprising, as active ingredient, a substituted quinoline or hydroquinoline compound or a pharmaceutically acceptable acid addition salt thereof as claimed in any one of claims 1 to 6 in association with a pharmaceutically acceptable diluent or carrier.

0107938

European Patent Office

# EUROPEAN SEARCH REPORT

Application number

EP 83 30 6052

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| Y | DE-A-3 039 848 (JUNEK)<br>* Claim 8; page 8, lines 1-15 *<br>--- | 1,8 | C 07 D 215/20<br>C 07 D 215/22<br>C 07 D 215/26<br>C 07 D 215/48<br>C 07 D 215/50<br>C 07 D 215/54<br>C 07 D 215/56<br>A 61 K 31/47 //<br>C 07 D 407/12<br>C 07 D 215/32 |
| Y | EP-A-0 025 864 (CASSELLA)<br>* Claim 1; page 6, lines 12-29; page 12, lines 4-21; pages 13,14 *<br>--- | 1,8 | |
| D,Y | EP-A-0 042 299 (KOWA)<br>* Whole document *<br>--- | 1,8 | |
| D,Y | EP-A-0 034 461 (KOWA)<br>* Whole document *<br>----- | 1,8 | |
| | | | TECHNICAL FIELDS SEARCHED (Int. Cl. 3) |
| | | | C 07 D 215/00<br>A 61 K 31/00 |

The present search report has been drawn up for all claims

| Place of search | Date of completion of the search | Examiner |
|---|---|---|
| THE HAGUE | 11-01-1984 | ALFARO I. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82